(19) Europäisches Patentamt
European Patent Office
Office européen des brevets

(11) EP 4 134 074 A1

(12) **EUROPEAN PATENT APPLICATION**
published in accordance with Art. 153(4) EPC

(43) Date of publication:
**15.02.2023 Bulletin 2023/07**

(51) International Patent Classification (IPC):
**A61K 9/72** (1974.07)       **A61K 9/14** (1974.07)
**A61K 31/196** (2000.01)       **A61P 31/16** (2000.01)

(21) Application number: **21783838.2**

(86) International application number:
**PCT/CN2021/080768**

(22) Date of filing: **15.03.2021**

(87) International publication number:
**WO 2021/203914 (14.10.2021 Gazette 2021/41)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **10.04.2020  CN 202010279686**

(71) Applicant: **Guangzhou Nucien Pharmaceutical
Co., Ltd.
Guangzhou, Guangdong 510530 (CN)**

(72) Inventors:
• **MIAO, Dong
  Guangzhou, Guangdong 510530 (CN)**
• **HU, Shuanghua
  Guangzhou, Guangdong 510530 (US)**

(74) Representative: **Becker, Eberhard
  Becker Kurig & Partner
  Patentanwälte mbB
  Bavariastraße 7
  80336 München (DE)**

(54) **PERAMIVIR DRY POWDER INHALANT AND PREPARATION METHOD THEREFOR**

(57)    The present invention relates to a peramivir dry powder inhalant, which is prepared from peramivir or an acceptable salt or a hydrate thereof. A single-dose formulation is 5-30 mg, the particle size D10 of dry powder is 1.3-2.2 um, D50 is 3-6 um, and D90 is 6-13 um. The peramivir dry powder inhalant can effectively reduce the titer of influenza A viruses in the lungs of a mouse, has a significant anti-virus effect, can significantly prolong survival time and reduces the death rate, and the pharmacodynamic effect of the peramivir dry powder inhalant is superior to those of a peramivir sodium chloride injection and oseltamivir phosphate. The peramivir dry powder inhalant has a specific lung targeting effect, and the effectiveness and safety of the medicine are significantly improved.

EP 4 134 074 A1

**Description**

FIELD

Embodiments of the present disclosure relate to pharmaceutical technologies, and more particularly relate to a peramivir dry powder inhaler (DPI) and a method of preparing the same.

BACKGROUND

Peramivir, the chemical name of which is (-)-(1S, 2S, 3R, 4R)-2- hydroxy-3-[(1S)-1-acetamido-2-ethyl]butyl-4-guanidinocyclopenta-1-carboxylic acid, is a cyclopentane derivative neuraminidase (NA) inhibitor against influenza virus.

Owing to its low oral availability, peramivir is mainly formulated into parenteral preparations such as injection. Peramivir may reside in a patient's respiratory system to act against influenza. Therefore, an inhaler form of peramivir, which is directly delivered to respiratory system lesions through a parenteral route, can mitigate potential adverse effects on other tissues of the whole body. An inhaler can exist in various dosage forms, including dry-powder inhaler, spray, solution inhaler, etc. In the patent application No. CN109771398B filed by the applicant of the present application, there is provided a peramivir solution inhaler, which discloses that if the dry powder inhaler is applied, it would be difficult for children or aged population, or those patients with spontaneous breathing difficulties such as respiratory function deficits to inhale effective amount of medication, while the solution inhaler is more appropriate to such patients. However, studies also reveal that the solution preparation faces a stability issue, while a dry powder inhaler offers a more stable formulation and a broader dosage range, as well as being convenient to carry; therefore, the present application focuses on developing a solid peramivir inhaler.

Peramivir plays an active role in preventing and treating avian influenza. Currently, the Covid-19 pandemic poses an unprecedented challenge to the public health systems around the world, while a directlydelivered, fast-acting and efficient medicine is still in need. With evolution of viruses, increasing obscurity of the boundary between habitats of human and animals, highly urbanization, more frequent interpersonal interactions, developed transportation networks, and agricultural intensification, viral epidemics such as avian influenza might occur more frequently and pose challenges in the future. Therefore, to prevent impact of the avian influenza upon the public health systems, the inventors of the present application focus on developing a solid inhalation preparation to provide a more efficient and effective solution.

MIU Xu et al. reports that internal cohesion and adhesive force are main action forces which affect the nebulization and dispersion properties of particles; these action forces depend on various factors, e.g., intrinsic properties of powder (surface energy, chemical group), particle characteristics (particle size distribution, morphology, roughness, porosity), and ambient conditions (mechanical process, temperature, and relative humidity), etc. A major issue that has been restricting development of preparations delivered to the lungs is drug loss before reaching the lungs. To achieve a desired clinical therapeutic effect through pulmonic drug delivery, the following aspects need to be taken into consideration: (1) preparation of drug powder, (2) whether to add a carrier; (3) nebulization of powder and delivery of the powder to the lungs via an inhaler. All of the above factors would affect pulmonary deposition and therapeutic effect of drug particles in the lungs.

WANG Xiaobo et al. reports that dry powder inhalers may be classified into four types: carrier-free, drug-carrier, drug-additive, and drug-carrier-additive, while the particle size, morphology, and density of particles are all important influencing factors. Particles have various morphologies, e.g., spherical shape, needle shape, polygonal shape, dendritic shape, fiber shape, and flake shape, etc. If a particle's morphology is irregular or far from a spherical shape, its aerodynamic behavior will be significantly affected. It is generally believed that spherically-shaped aerosol particles have a better aerodynamic behavior. Particle density is also an important factor affecting the pulmonary deposition property of the dry powder inhaler. In addition, good dispersion property of powder could facilitate nebulization" into inhalable drug particles, which improves the accuracy of inhaled dosage. The main factors affecting powder flowability and dispersibility include: 1. Van der Waals force, which can be significantly affected by particle size, surface roughness, geometric morphology, and particle deformation; 2. electrostatic force, wherein the static produced by inter-particle friction during the dry powder inhaler manufacturing process would affect the micromeritic properties such as flowability, dispersity, and adhesivity of the powder, thereby affecting its formulation, production and use; 3. capillary force: due to hygroscopicity of particles, surface tension and capillary attraction are produced when moisture is adsorbed on particle surfaces, whereby affecting particle size and crystalline morphology, causing powder agglomeration, and decreasing dispersity of the powder; 4. shear force between particles with irregular morphologies; 5. friction force produced by relative movement between particles.

Adding a carrier offers the following advantages: improving powder flowability and enhancing pulmonary deposition property of the medication. By increasing the single-dose volume, accurate subpackaging of small-dosage drugs is realized, which improves reproductivity of the inhaler drug's delivered dose. However, adding the carrier also incurs some problems, for example, the medicated fine powder has a very strong surface adsorption property with the carrier such that the medication cannot be detached from the carrier after being inhaled and would be deposited at the oropharynx together with the carrier, which decreases the amount of effective medicine entering the respiratory tract and thus degrades the pulmonary deposition property.

Another additive, a very fine powder such as leucine and phospholipid, may be added to the drug powder, which can significantly improve flowability of the dry powder inhaler. However, in some cases, addition of the very fine powder does not improve powder flowability much, because drug powder type, very fine powder type, particle size, surface roughness, particle deformation, and gas adsorption may all have an impact on the interaction between particles.

TIAN Pei et al. reports that nebulization of a dry powder medicine is a process influenced by multiple factors, which is not only influenced by carrier type, particle size, morphology, and surface charge, but also influenced by carrier surface roughness and crystallinity.

LI Zhiwan et al. reports that triboelectricity between particles or between particles and the inner wall of the device occurs commonly to powder aerosols. The electrostatic phenomenon is complex and also difficult to study and control. This is because the charges carried by the dry powder to be inhaled are related to physicochemical properties of the powder, design of the inhaler device, and ambient factors, which are also a result of joint actions of a plurality of factors. Therefore, even a slight change of the physicochemical properties of the powder or a change of methodology would significantly affect the electrostatic behavior of the dry powder to be inhaled.

ZHU Wanhui et al. reports that the macroscopic properties such as flowability and dispersity depend on relative strength of the inter-particle cohesion (drug/drug) and adhesion (drug/carrier). The inter-particle action force must be strong enough to maintain stability of the preparations in storage and use and allow for drug decohesion before or during inhalation. The relative strength of various inter-particle action forces (mainly the Van de Waals force, capillary force, electrostatic action force, and mechanical crosslinking induced by surface roughness) are determined by material properties, ambience, and processing conditions of the drug, carrier, and additive. During the preparation process, a balance must be achieved between blend stability during storage and processing and the dispersion process during inhalation, which requires control of the inter-particle action forces. Process-related variables will influence each other in different ways. If one variable changes, influence from the other variables may lead to a reverse result. This is why some literatures on single-variable influence led to a reversed conclusion.

To improve particle properties, a conventional practice is to add a carrier and/or additive to a dry powder inhaler. However, the inhalation effect of the dry powder inhaler is affected comprehensively by various factors, while the various factors would influence one another, such that when one factor changes, the influence of other factors would lead to a reverse result. Therefore, development of a clinically effective pharmaceutical dry powder inhaler still faces various technical challenges.

SUMMARY

To overcome the above and other drawbacks in conventional technologies, the present disclosure provides a novel form of peramivir inhaler preparation, i.e., a solid dry powder inhaler preparation which is carrier-free, expedient-free, and additive-free. The dry powder of the peramivir inhaler preparation has a uniformly distributed particle size range, morphology, and flowability suitable for pulmonary inhalation. The dry powder is hardly agglomerated, which can effectively improve single dose and has an appropriate pharmaceutical stability. Another aspect of the present disclosure further provides a more effective method of preparing a dry powder, which obtains a micropowder with a particular particle size and morphology by jet milling a peramivir trihydrate, whereby effective pulmonary delivery of medicine is achieved.

A technical solution for solving the above technical problems is provided below:

A peramivir dry powder inhaler, comprising: dry powder of a peramivir active pharmaceutical ingredient or its acceptable salt or hydrate, wherein a single-dose preparation of the dry powder inhaler has a content ranging from 5mg to 30mg; particle size distribution D10 of the dry powder ranges from 1.3$\mu$m to 2.2$\mu$m, particle size distribution D50 ranges from 3$\mu$m to 6$\mu$m, and particle size distribution D90 ranges from 6$\mu$m to 13$\mu$m.

The dry powder has an angle of repose ranging from 33° to 37°, preferably 34°, 35°, 36°; a bulk density ranging from 0.23g/cm$^3$ to 0.28g/cm$^3$, preferably 0.24, 0.25, 0.26, 0.27g/cm$^3$; a tapped density ranging from 0.39 to 0.44g/cm$^3$, preferably 0.40, 0.41, 0.42, 0.43g/cm$^3$.

The Carr's index of the dry powder ranges from 58 to 65, preferably 59, 60, 61, 62.

The percentage of the dry powder with a fine powder fraction FPF less than 4.46$\mu$m is greater than 30% and less than 45%, preferably 32%, 33%, 34%, 35%, 36%, 37%, 38%, 40%, 41%, and more preferably ranging from 34% to 38%.

The dry powder has an aerodynamic particle size of greater than 2.5$\mu$m and less than 3.6$\mu$m, preferably ranging from 2.8$\mu$m to 3.5$\mu$m or from 2.6$\mu$m to 3.1$\mu$m or from 2.9$\mu$m to 3.4$\mu$m, and more preferably 3.0$\mu$m to 3.2$\mu$m or from 3.1$\mu$m to 3.3$\mu$m.

The dry powder has a particle size distribution D10 ranging from 1.3$\mu$m to 2.5$\mu$m, preferably 1.4$\mu$m, 1.5$\mu$m,

1.6μm, 1.7μm, 1.8μm, 1.9μm, 2.1μm, 2.2μm, 2.3μm, and 2.4 μm; a particle size distribution D50 of preferably 3.5μm, 4.5μm, 5μm, 5.5μm; and a particle size distribution D90 of preferably 6.2μm, 6.5μm, 7μm, 7.5μm, 7.8μm, 8.0μm, 8.4μm, 9.0μm, 10μm, 11μm, 12μm.

The dry powder inhaler is prepared by a peramivir trihydrate.

The present disclosure further provides a single-dose peramivir dry powder inhaler preparation, wherein the dry powder described above is prepared into a capsule dosage form, a niosome dosage form, or a depot dosage form, a dry powder loading capacity of each unit of dosage form ranges from 5 to 30mg, preferably 20mg.

Capsule dosage form is preferred which is prepared by adding the dry powder into a hydroxypropyl methyl cellulose HPMC capsule shell, wherein the dry powder in each capsule has a content ranging from 10 to 30mg, preferably 20mg.

The peramivir dry powder inhaler preparation further comprises a carrier and/or an excipient, or is carrier-free and/or excipient-free. More preferably, the peramivir dry powder inhaler preparation is carrier-free and/or excipient-free.

The carrier and/or expedient is selected from one or more of lactose, mannitol, leucine, and phospholipid. The disclosure further provides a method of preparing a peramivir dry powder inhaler, comprising: jet milling a peramivir trihydrate to obtain a dry powder at a feed rate 120~140V, a feed pressure 0.4~0.55MPa, preferably 0.42, 0.45, 0.48, 0.5, 0.52, 0.55MPa, and a milling pressure 0.4~0.6MPa, preferably 0.42, 0.45, 0.5, 0.52, 0.55, 0.58MPa.

The present disclosure further provides a method of preparing a peramivir dry powder inhaler drug, comprising: loading the dry powder obtained from the above method and an appropriate excipient into a hydroxypropyl methyl cellulose HPMC capsule shell, wherein the dry powder and the appropriate excipient are homogeneously blended or treated by an appropriate process to obtain a blend micropowder, and the blend micropowder is added into each capsule, a content of micropowder in which is 10~30mg, preferably 20mg.

The present disclosure further provides a peramivir dry powder inhaler, comprising: peramivir or an acceptable salt or hydrate thereof, and mannitol, wherein the mass ratio between the pharmaceutical active ingredient and the mannitol is 1:1 ~1:5, preferably 1:1, 1:2. The peramivir dry powder inhaler preferably comprises a blend of a spray dry powder of the peramivir or an acceptable salt or hydrate thereof and mannitol. The dry powder has a $\rho$bulk density (g/cm$^3$) ranging from 0.13 to 0.35, preferably 0.14, 0.18, 0.20, 0.23, 0.25, 0.28, and 0.3, a $\rho$tapped density (g/cm$^3$) ranging from 0.13 to 0.36, preferably 0.14, 0.18, 0.20, 0.23, 0.25, 0.28, 0.3, 0.33, a median particle size distribution $D_g$ (μm) ranging from 3.4 to 9μm, preferably 3.5, 3.6, 3.8, 4.0, 4.4, 4.6, 4.8, 5.0, 5.2, 5.5, 6.0, 6.5, 7.0, 7.4, 7.8, 8.0, 8.5, 9.0μm, an aerodynamic particle size distribution $D_a$ (μm) ranging from 1.8 to 6μm, preferably 2.0, 2.2, 2.4, 2.6, 2.8, 3.0, 3.2, 3.4, 3.6, 3.8, 4.0, 4.4, 4.8, 5.2, 5.4, 5.6, 5.8μm, and an angle of repose ranging from 29° to 35°, preferably 30°, 31°, 32°, 33°, 34°.

The disclosure further provides a peramivir dry powder inhaler, comprising a peramivir or its acceptable salt or hydrate, and mannitol, wherein the mass ratio between the pharmaceutical active ingredient and the lactose is 1:1 ~ 1:4, preferably 1:1, 1:2. The peramivir dry powder inhaler is preferably prepared by a blend of jet milled powder of the peramivir or its acceptable salt or hydrate and the lactose. The lactose is selected from Inhalac 120 or Inhalac 400, more preferably Inhalac 120. The dry powder inhaler has a $\rho$bulk density (g/cm$^3$) ranging from 0.3 to 0.5, preferably 0.32, 0.36, 0.4, 0.45, 0.5, a $\rho$tapped density (g/cm$^3$) ranging from 0.5 to 0.7, preferably 0.55, 0.56, 0.58, 0.6, 0.62, 0.64, 0.66, 0.68, an aerodynamic particle size distribution Da (μm) ranging from 5.5 to 6.5μm, preferably 5.2, 5.4, 5.6, 5.8, 6.0, 6.2μm, and an angle of repose ranging from 34° to 40°, preferably 34.5°, 35°, 35.5°, 36°, 37°, 38°.

The disclosure offers the following benefits:

1. The method of preparing a peramivir dry powder inhaler according to the disclosure is easy to operate, which ensures efficacy and safety of the drug prepared.

2. The peramivir dry powder inhaler has an appropriate particle size distribution range, morphology, and powder flowability; the dry powder is not prone to agglomerate, which can effectively reduce single dose and has an appropriate pharmaceutical stability.

3. The peramivir dry powder inhaler according to the present disclosure is prepared by jet milling a peramivir trihydrate, which can obtain a micropowder with a particular particle size and morphology, whereby to achieve effective pulmonary delivery of medicine.

4. The peramivir dry powder inhaler according to the present disclosure can effectively mitigate influenza A virus titer in lungs of mice, has a notable antiviral effect, can significantly extend survival time of the mice, and lowers the mortality rate; its therapeutic effect is superior to peramivir sodium chloride injections and oseltamivir phosphate.

DETAILED DESCRIPTION

Hereinafter, the present disclosure will be further described with reference to the accompanying drawings. It is understood that the embodiments provided herein are only intended for illustrating the present application, not intended for limiting the scope of the present disclosure. The experiment methods without setting forth specific conditions in the embodiments follow conventional conditions or those recommended by manufacturers.

Unless otherwise defined, all professional and scientific terminologies used herein have meanings familiar to those skilled in the art. In addition, any method and material similar or equivalent to the contents disclosed herein may be applied to the method of the present disclosure. The preferred implementation methods and materials are only for illustrative purposes.

The present disclosure involves the following determination methods:

1. determining the angle of repose, comprising: determining the angle of repose by a fixed funnel method, wherein the drug powder flew out from the funnel at a predetermined velocity; then the diameter (D) and height (h) of the piled powder were measured, wherein r=D/2; then the angle of repose was calculated according to the equation *tana*=h/r.

2. determining the bulk density and tapped density, comprising: uniformly transferring the drug powder into a measuring cylinder and tapping the drug powder therein 100 times, wherein the pre-tapping drug weight and volume and the post-tapping drug weight and volume were recorded.

3. determining the particle size of the powder, comprising: placing the drug powder on a laser particle size analyzer to measure its particle size.

$D_g$ and aerodynamic particle size $D_a$: the median particle size ($D_g$) was measured; the aerodynamic diameter ($D_a$, MMAD) was calculated according to the equation:

$$D_a = D_g \sqrt{\frac{\rho}{\rho_0 \chi}}$$

.

## 1. Example 1

The peramivir spray dry powder formulations were set forth below:

Table 1: Peramivir Spray Dry Powder Formulations

| Ingredients | Composition 1 | Composition 2 |
|---|---|---|
| peramivir | 6g | 6g |
| leucine | 0 | 600mg |
| phospholipid | 0 | 120mg |
| ultrapure water | 120ml | 120ml |

Process conditions: the parameters of spray drying process were as such: inlet temperature 200°C, outlet temperature 100°C, and velocity: 400ml/h. The resulting powder was sieved through a 180-mesh sieve. The experiment results were set forth below:

Table 2: Experiment Results of Peramivir Spray Dry Powder (n=3)

| Indexes | Composition 1 | Composition 2 |
|---|---|---|
| $\rho$bulk density(g/cm$^3$) | 0.080 | 0.08 |
| $\rho$tapped density(g/cm$^3$) | 0.144 | 0.14 |
| median particle size $D_g$ ($\mu$m) | 3.29 | 2.99 |
| aerodynamic particle size $D_a$ ($\mu$m) | 1.25 | 1.12 |
| angle of repose $\alpha$(°) | 38.81 | 44.86 |

The experiment results show that after the formulation was optimized by adding phospholipid and amid acid, the density and particle size of the spray dry powder were diminished, but flowability was deteriorated.

2. The spray dry powder was blended with mannitol to obtain a dry powder blend, wherein the peramivir spray dry powder prepared according to composition 1 was sufficiently blended with the mannitol in proportions of: 1:1; 1:2; 1:5(w/w). The experiment results are set forth below:

Table 3 Experiment Results of the Peramivir Spray Powder and Mannitol Blends(n=3)

| Indexes | Composition 1 | Composition 1: Mannitol (1:1) | Composition 1: Mannitol (1:2) | Composition 1: Mannitol (1:5) |
|---|---|---|---|---|
| $\rho$bulk density (g/cm$^3$) | 0.080 | 0.140 | 0.224 | 0.313 |
| $\rho$tapped density (g/cm$^3$) | 0.144 | 0.144 | 0.293 | 0.322 |
| median particle size $D_g$ ($\mu$m) | 3.29 | 3.693 | 4.732 | 8.457 |
| aerodynamic particle size $D_a$ ($\mu$m) | 1.25 | 2.000 | 2.650 | 5.733 |
| angle of repose $\alpha$ (°) | 38.81 | 33.62 | 29.40 | 31.54 |

The experiment results show that after the peramivir dry powder was blended with mannitol, the flowability improves although the particle size and density increase prominently.

3. The spray dry powder was blended with lactose to obtain a dry powder blend, wherein the peramivir spray dry powder of compositions 1 was blended with lactose in a proportion of 1:1 (w/w).

Table 4 Experiment Results of the Peramivir Spray Dry Powder and Lactose Blend (n=3)

| Indexes | Composition 1 | Composition 1: Lactose (1:1) |
|---|---|---|
| $\rho$bulk density (g/cm$^3$) | 0.080 | 0.15 |
| $\rho$tapped density (g/cm$^3$) | 0.144 | 0.32 |
| median particle size $D_g$($\mu$m) | 3.29 | 6.07 |
| aerodynamic particle size $D_a$($\mu$m) | 1.25 | 2.05 |
| angle of repose $\alpha$ (°) | 38.81 | 44.0 |

The experiment results show that after the dried powder was blended with lactose (1:1, w/w), its density and particle size increased, but its flowability was deteriorated.

It was also found in the experiment that the electrostatic adsorption between spray dried powder particles increased, such that the dry powder was easily agglomerated. Therefore, the spray dried peramivir powder was not suitable for being prepared into a dry powder inhaler, and this situation is not improved even after blending with lactose.

**2. Example 2**

Formulation 1: peramivir dry micropowder, which was prepared by jet milling peramivir active pharmaceutical ingredient (API) under the following conditions: feed rate 130V, feed pressure 0.45MPa, and milling pressure 0.45MPa.

Formulation 2: the dry powder resulting from Formulation 1 was blended with lactose Inhalac 120 in a mass ratio of 1:1 to obtain a powder blend.

Formulation 3: the dry powder resulting from Formulation 1 was blended with lactose Inhalac 400 in a mass ratio of 1:1 to obtain a powder blend.

**Pulmonary Deposition Ratio Experiment**

The powders resulting from Formulations 1 to 3 were loaded into capsules, respectively, 20mg for each capsule.

Before starting the experiment, the NGI (Next Generation Pharmaceutical Impactor) was closed and an induction port was installed and tightly sealed with a seal film. The experiment procedure comprised: first turning on the pump, turning on the flow meter, turning on the TPK(critical flow controller) instrument; then connecting the flow meter to the induction port, loading empty capsules and punching holes through the capsules with a pen-type drug delivery device, and connecting the pen-type drug delivery device to the flow meter to measure the flow rate. Parameters of the DPI particle size distribution analyzer were set as such: Flow = 60; P3= 14.4; P2=66.8. The number of sample capsules fed was set to 20, and the feed duration was set to 7 seconds. Then, an adaptor was installed to the induction port. The drug-loaded capsules were connected to the inhaler device of the drug particle size distribution analyzer; and then the inhalation button was clicked; the above operations were repeated twenty times. After the 20 capsules were completely inhaled, the drug powder inhaled in the collection trays of the induction port, stage 1, stage 2, stage 3, and stage 4 was rinsed twice with 10ml deionized water, respectively; the rinsed water was then transferred into a 50ml volumetric flask; meanwhile, the inhaler device and the induction port were rinsed with ultrapure water, and the rinsed water was collected into a volumetric flask, respectively. The drug powder inhaled in the collection trays of stage 6, stage 7, and stage 8 of the NGI was rinsed twice with 10ml deionized water, respectively; the rinsed ultrapure water was transferred in a 25ml volumetric flask. Finally, the fine particle fraction (FPF) of each formulation was measured. The results are set forth below:

Table 5 Fine Particle Fraction (n=3)

| Result | Formulation 1 | Formulation 2 | Formulation 3 |
|---|---|---|---|
| FPF,<4.46μm | 31.32±0.02 | 33.97±2.99 | 13.97±0.06 |

Table 6 Distribution of the Powder in Various Stages of the NGI (n=3)

| NGI Stages | Mean Content (mg) | | |
|---|---|---|---|
| | Formulation 1 | Formulation 2 | Formulation 3 |
| Stage 1 | 62.70 | 91.79 | 74.90 |
| Stage 2 | 32.14 | 35.64 | 41.32 |
| Stage 3 | 32.45 | 30.71 | 22.88 |
| Stage 4 | 31.76 | 31.93 | 15.00 |
| Stage 5 | 24.90 | 55.34 | 8.29 |
| Stage 6 | 13.95 | 14.26 | 2.81 |
| Stage 7 | 3.59 | 6.12 | 0.81 |
| Stage 8 | 3.25 | 3.97 | 0.62 |
| Inhaler Device | 118.90 | 109.96 | 21.39 |
| Induction Port | 16.92 | 27.61 | 168.27 |
| Total | 340.56 | 407.33 | 356.29 |

In terms of FPF, formulation 1 is approximate to formulation 2, but formulation 3 has a much smaller FPF, which is approximate to 10% of the criteria provided in the pharmacopoeia. The particles in formulation 3 were mainly stuck at the induction port, a possible cause of which is that too fine lactose particles easily produce static. It is seen that formulation 3 is not suitable as a peramivir dry powder formulation.

**Stability Experiment**

Capsules with formulations 1 and 2 were placed for 0 days, 5 days, and 10 days under 60°C, respectively, to observe appearance change of the blends and measure the following indexes:
(1) Delivered Dose Uniformity (DDU)
Capsules with formulations 1 and 2 were connected to a DDU device, wherein the sample loaded in each capsule had a content of 20mg; the pump was turned on, the flow meter was connected to a collection tube, wherein one end of the collection tube was placed with filter paper, and the other end thereof was connected to the flow meter; a DUSA (Dosage Unit Sampling Apparatus) collection tube was connected to the flow meter; and empty capsules were loaded and punched with holes with the pen-type drug delivery device to measure the flow rate. The TPK (critical flow controller) instrument was turned on; the Set Up button on the TPK instrument was pressed to set parameters; after the parameters were set, the "OK" button was pressed, and then the "set P1" button was pressed to adjust the flow rate to 60L/min. The "set flow" button was pressed, and when the screen displays flow (Q), the "Yes" button was pressed; and then the "Yes" button was pressed again; the readings on the screen were recorded, and then the flow meter was removed. The DDU parameters were set as such: Flow = 60; P3 = 14.4; P2=66.8. The drug-loaded capsules were placed in an inhaler device manufactured by Suzhou Wantong; the inhalation button was clicked, with 1 capsule being inhaled each time. The medicine on the filter paper and in the collection tube was rinsed with ultrapure water into a 50ml beaker; the beaker was rinsed three times, and the collected liquid was transferred into a 50ml volumetric flask, and then ultrapure water was dripped into the flask to bring to volume. 10 parts of collected liquid were subjected to HPLC detection to determine the content of drug in each part of the collected liquid.

(2) Fine Particle Fraction (FPF<4.46$\mu$m), which was measured by NGI.

Table 7 Experiment Results of DPI Stability under High Temperature (60°C)

| formulat ions | results | | | | | | | | |
|---|---|---|---|---|---|---|---|---|---|
| | Day 0 | | | Day 5 | | | Day 10 | | |
| | Appeara nce | FPF<4.4 6$\mu$m | Delivered dose% | appeara nce | FPF<4.4 6$\mu$m | Delive red dose% | appeara nce | FPF<4.4 6$\mu$m | Delive red dose% |
| Formulat ion 1 | White powder | 27.18±0. 03 | 99.99 ±16.02 | White powder | 27.63±0. 16 | 100.02 ±10.82 | White powder | 26.00±0. 59 | 105.54 ±13.30 |
| Formulat ion 2 | White powder | 32.37±2. 82 | 101.82±1 6.87 | White powder | 21.82±0. 08 | 105.70 ±22.88 | White powder | 18.43±0. 27 | 100.05 ±2.40 |

The above table shows the delivered doses of different formulations under different influencing factors, wherein the samples (20mg per capsule) were manually loaded and accurately quantified. The results show that the delivered doses meet the criteria (mean value between 75% and 125%) specified in the pharmacopeia, and high temperature does not affect the DDU much.

The fine particle fraction (FPF<4.46$\mu$m) results show that the high temperature does not affect the FPF of formulation 1 much, but affects the FPF of formulation 2 much. Therefore, additive-free, carrier-free peramivir is the optimal formulation, while blend with excipients and carriers does not lead to performance improvement.

**Example 3**

A 1.3kg peramivir trihydrate API was milled with a jet mill to obtain a dry powder under feed rate 130V, inlet pressure 0.45Mpa, and milling pressure 0.40MPa; the dry powder was loaded into HPMC (Hydroxypropyl Methyl Cellulose) capsules, 20mg for each capsule, wherein the particle size distribution D10 of the dry powder was 1.934$\mu$m, D50 was 4.415$\mu$m, and D90 was 8.858$\mu$m; the angle of repose was 34.8°;the bulk density was 0.25g/cm$^3$, the tapped density was 0.41g/cm$^3$, and the Carr's index was 59.0.

**Example 4**

A 1.3kg peramivir trihydrate ingredient was milled with a jet mill to obtain a dry powder under feed rate 125V, inlet pressure 0.50Mpa, and milling pressure 0.45MPa; the dry powder was loaded into HPMC (Hydroxypropyl Methyl Cellulose) capsules, 20mg for each capsule, wherein the particle size distribution D10 of the dry powder was 1.447$\mu$m, D50 was 3.269$\mu$m, and D90 was 6.988$\mu$m. The results showed that the angle of repose was 35.3°, the bulk density was 0.26g/cm$^3$, the tapped density was 0.43g/cm$^3$, and the Carr's index was 60.0.

**Example 5**

A 1.3kg peramivir trihydrate ingredient was milled with a jet mill to obtain a dry powder under feed rate 135V, inlet pressure 0.42Mpa, and milling pressure 0.40MPa; the dry powder was loaded into HPMC (Hydroxypropyl Methyl Cellulose) capsules, 20mg for each capsule, wherein the particle size distribution D10 of the dry powder was 2.034$\mu$m, D50 was 5.716$\mu$m, and D90 was 12.67$\mu$m. The results showed that the angle of repose was 34.1°, the bulk density was 0.23g/cm$^3$, the tapped density was 0.39g/cm$^3$, and the Carr's index was 61.0.

**Example 6**

A 1.3kg peramivir trihydrate ingredient was milled with a jet mill to obtain a dry powder under feed rate 140V, inlet pressure 0.40Mpa, and milling pressure 0.35MPa; the dry powder was loaded into HPMC (Hydroxypropyl Methyl Cellulose) capsules, 15mg for each capsule, wherein the particle size distribution D10 of the dry powder was 2.89$\mu$m, D50 was 7.78$\mu$m, and D90 was 16.39$\mu$m. The results showed that the angle of repose was 33.7°, the bulk density was 0.24g/cm$^3$, the tapped density was 0.41g/cm$^3$, and the Carr's index was 65.0.

**Example 7**

A 1.3kg peramivir trihydrate ingredient was milled with a jet mill to obtain a dry powder under feed rate 120V, inlet pressure 0.60Mpa, and milling pressure 0.40MPa; the dry powder was loaded into HPMC (Hydroxypropyl Methyl Cellulose) capsules, 15mg for each capsule, wherein the particle size distribution D10 of the dry powder was 1.332$\mu$m, D50 was 3.015$\mu$m, and D90 was5.658$\mu$m. The results showed that the angle of repose was 38.4°, the bulk density was 0.28g/cm$^3$, the tapped density was 0.47g/cm$^3$, and the Carr's index was 66.0.

**Experiment Example 1**

**Inhaled Powder NGI Delivery Experiment**

1. Preparing Samples

The dry powder sample obtained from Example 1 was subjected to NGI delivery test to measure its *in vitro* deposition ratio. The sample was filled in single-dose capsules. The test was performed three times, 10 capsules delivered each time.

2. NGI Test Results

Upon completion of the NGI delivery test, the sample in each reception cup/ sample tray of the NGI was rinsed, respectively; the rinsed water was collected into a volumetric flask and brought to volume; and then the content was measured using the HPLC method.

Table 8 *In Vitro* Deposition Ratio Test Results of Example 1

| Batch No. | | Example 3 | |
|---|---|---|---|
| Number of Samples under Test | | 10 capsules per time | |
| Number of Times of Testing | | 3 times | |
| Flow Rate | | 60 L/min | |
| Distribution of Samples at Various Stages of NGI | | Software Calculation Results of NGI Delivery *In Vitro* Deposition Ratio | |
| Inhaler Device | 17.83% | Metered(mg) | 20.689 |
| Induction Port | 32.86% | Delivered Dose (mg) | 16.998 |
| Preseparator | 9.43% | Fine particle dose (mg) | 6.129 |
| Stage 1 | 3.75% | FPF(%) | 36.038 |
| Stage 2 | 8.62% | MMAD(um) | 3.271 |
| Stage 3 | 11.37% | GSD | 1.961 |
| Stage 4 | 8.58% | R^2 | 0.993 |
| Stage 5 | 3.72% | | |
| Stage 6 | 2.02% | | |
| Stage 7 | 1.14% | | |
| MOC (Micro-Orifice Collector) | 0.66% | | |
| Stages 2-7 | 35.46% | | |
| Stages 3-7 | 26.84% | | |

**Experiment Example 2**

**Comparative Experiment on *In vitro* Deposition Ratios of Different Particle Sizes**

The samples obtained from Examples 1 to 4 were subjected to NGI *in vitro* deposition ratio test. The testing results are set forth below:

Table 9 Results of NGI *In Vitro* Deposition Ratio Test on Different Particle Sizes

| Batch No. | Example 3 | Example 4 | Example 5 | Example 6 |
|---|---|---|---|---|
| Number of Samples under Test | 10capsules per time | 10capsules per time | 10capsules per time | 10capsules per time |
| Number of Testing | 3 | 3 | 3 | 3 |
| Flow Rate | 60 L/min | 60 L/min | 60 L/min | 60 L/min |
| Inhaler Device | 17.84% | 24.89% | 17.86% | 18.97% |
| Induction Port | 32.86% | 18.12% | 40.94% | 55.37% |
| Preseparator | 9.43% | 9.19% | 8.32% | 9.56% |
| Stage 1 | 4.05% | 2.57% | 3.19% | 4.93% |

(continued)

| Batch No. | Example 3 | Example 4 | Example 5 | Example 6 |
|---|---|---|---|---|
| Stage 2 | 8.62% | 8.20% | 6.78% | 5.01% |
| Stage 3 | 11.37% | 13.30% | 8.75% | 4.12% |
| Stage 4 | 8.58% | 14.24% | 8.19% | 1.35% |
| Stage 5 | 3.72% | 6.47% | 3.71% | 0.43% |
| Stage 6 | 2.02% | 2.16% | 1.58% | 0.11% |
| Stage 7 | 1.14% | 21.38% | 0.49% | 0.09% |
| MOC | 0.66% | 0.26% | 0.20% | 0.06% |
| Stages 2-7 | 35.46% | 65.76% | 29.50% | 11.17% |
| Stages 3 ~7 | 26.84% | 57.56% | 22.71% | 6.16% |

The *in vitro* deposition ratio of samples with a particle size D90 greater than 12.67μm can hardly meet the criteria specified in the pharmacopeia (which requires a fine particle dose not less than 10%), while Example 2 provides a good *in vitro* deposition ratio.

**Experiment Example 3**

**Stability Experiment**

The dry powder obtained from Example 1 was exposed to high-temperature (60°C) and sampled on day 0, day 5, day 10, and day 30 to test their NGI *in vitro* deposition ratio.

Table 10 Stability Experiment on the Dry Powder of Example 1 under High Temperature (60 °C)

| Batch No. | Items under Test | Starting sample | Capsule | | |
|---|---|---|---|---|---|
| | | Dav 0 | Dav 5 | Day 10 | Day 30 |
| Sample 1 | Metered(mg) | 20.689 | 20.309 | 21.608 | 21.695 |
| | Delivered(mg) | 16.998 | 16.179 | 16.488 | 17.378 |
| | Fine Particle Dose(mg) | 6.129 | 6.306 | 6.473 | 5.780 |
| | FPF(%) | 36.038 | 38.978 | 39.219 | 33.274 |
| | MMAD(μm) | 3.271 | 3.451 | 3.496 | 3.182 |
| | GSD | 1.961 | 1.945 | 1.893 | 2.007 |
| | R^2 | 0.993 | 0.995 | 0.999 | 0.980 |
| | Stage 1 | 3.75% | 4.44% | 4.02% | 3.35% |
| | Stage 2 | 8.62% | 10.60% | 10.59% | 7.60% |
| | Stage 3 | 11.37% | 12.05% | 12.01% | 9.51% |

(continued)

| Day 0 | Day 5 | Day 10 | Day 30 | | |
|---|---|---|---|---|---|
| | Stage 4 | 8.58% | 8.87% | 9.46% | 6.84% |
| | Stage 5 | 3.72% | 3.64% | 3.85% | 3.32% |
| | Stage 6 | 2.02% | 2.02% | 1.29% | 2.55% |
| | Stage 7 | 1.14% | 1.12% | 0.43% | 1.53% |
| | Stage 8 | 0.65% | 0.74% | 0.26% | 1.02% |
| | Induction Port | 32.86% | 28.24% | 26.54% | 35.98% |
| | Inhaler Device | 17.83% | 20.35% | 23.69% | 19.90% |
| | Preseparator | 9.43% | 7.94% | 7.85% | 8.42% |
| | Deposition Ratios of Stages 3～7 | 26.84% | 27.70% | 27.04% | 23.7% |
| | Deposition Ratios of Stages 2～7 | 35.46% | 38.30% | 37.64% | 31.3% |

The experiment results show that the dry powder inhaler according to the present disclosure has a superb stability at a high temperature.

Experiment Example 4

*In Vivo* Protection of Nebulized Peramivir Dry Powder Inhaler Administered to Mice Infected With Influenza A/Pr/8/34 Virus

80 SPF Balb/c mice, half male and half female, weighing 16.9~22.6g each, were stochastically divided into 8 groups: a normal control group, a virus model group, an oseltamivir phosphate capsule group (Tamiflu) (10mg/kg), a peramivir intravenous injection group (10mg/kg), and peramivir dry powder inhaler (example 3) groups I-IV (0.1, 0.3, 0.9, 2.7mg/kg), ten mice in each group. The mice in each group were mildly anaesthetized with diethyl ether and intranasally administered with influenza A/PA/8/34 virus liquid (5 $LD_{50}$ diluted solution resulting from diluting the influenza virus bulk with a 4°C precooled blank culture medium), 60$\mu$L per mouse, wherein the normal control group was intranasally administered with the blank culture medium of the same volume. Prior to daily administration, the peramivir intravenous injection was prepared into a 1.0mg/mL liquor with 0.9% sodium chloride injection, and the oseltamivir phosphate capsule was prepared into a 0.5mg/mL liquor with pure water. The mice in each group were all administered at 2h postmodeling of the intranasal virus infection, wherein the mice in each peramivir dry powder inhaler group were administered with a corresponding dosage of peramivir dry powder inhaler, the mice in the peramivir intravenous injection group were intravenously injected with the peramivir liquid as per a dosage of 10mL/kg, the oseltamivir phosphate capsule group were intragastrically administered with oseltamivir phosphate liquid as per a dosage of 20mL/kg, and the normal control group and the model control group were sprayed with air of the same volume via their trachea. The mice were administered once per day, 5 days in succession. The first day (D1) of virus infection was counted from the next day of the intranasal virus inflection modeling.

Starting from the day of infection modeling, the mice were observed for 15 days successively to record the weights, death time, and number of deaths of the mice in each group, so as to measure weight changes during the administration period and calculate the morality rate (= number of dead mice/total number of mice x 100%) and the average survival time (= total number of survival days of the mice/ number of mice).

Table 11 Protective Effect of Peramivir Against Death of Mice Infected with Influenza A/PR/834 Virus ($\overline{x}\pm s$)

| Group # | Dosage (mg/kg) | Number of mice | Number of deaths | Number of survivals | Mortality rate (%) | Average number of survival days |
|---|---|---|---|---|---|---|
| Normal control group | / | 10 | 0 | 10 | 0 | 15.0±0.0 |
| Virus control group | / | 10 | 10 | 0 | 100++ | 6.2±1.0++ |
| Oseltamivir phosphate group | 10 | 10 | 4 | 6 | 40** | 11.3±4.8* |

(continued)

| Group # | Dosage (mg/kg) | Number of mice | Number of deaths | Number of survivals | Mortality rate (%) | Average number of survival days |
|---|---|---|---|---|---|---|
| Peramivir I.V. injection group | 10 | 10 | 3 | 7 | 30** | 12.3±4.4** |
| Peramivir DPI group I | 0.1 | 10 | 7 | 3 | 70 | 8.7±4.4 |
| Peramivir DPI group II | 0.3 | 10 | 5 | 5 | 50** | 10.4±4.9 |
| Peramivir DPI group III | 0.9 | 10 | 2 | 8 | 20** | 13.2±3.8** |
| Peramivir DPI group V | 2.7 | 10 | 1 | 9 | 10** | 14.0±3.2** |
| Compared with the normal control group, ++$P \leq 0.01$; compared with the virus control group, $P<0.05$, **$P \leq 0.01$. | | | | | | |

As shown in Table 11, during the experiment period, the mice in the normal control group were not infected by virus such that no death occurred thereto; while 10 mice in total were dead in the 10-mice virus control group, the mortality rate amounting to 100%, which attained a significant statistical differnce ($P \leq 0.01$) over the normal control group. Compared with the virus control group, the mortality rates of the mice in the oseltamivir phosphate group, the peramivir intravenous injection (I.V.) group, and the peramivir DPI groups II-IV were significantly decreased ($P \leq 0.05$ or $P \leq 0.01$); while the peramivir DPI group I had no statistical significance ($P>0.05$) compared with the virus control group. Compared with the normal control group, the average number of survival days of the mice in the virus control group is 6.2, which is significantly decreased ($P \leq 0.01$). Compared with the virus control group, the average survival time of peramivir DPI groups I and II was prolonged to a certain extent, but they had no statistical difference ($P>0.05$). The average survival time of the oseltamivir phosphate group, the peramivir I.V. injection group, and the peramivir DPI groups III-IV were significantly prolonged ($P \leq 0.05$ or $P \leq 0.01$) over the virus control group.

The analysis of the results above shows that administration of the peramivir DPI at 2h post-infection can significantly prolong the survival time and lower the mortality rate, which has a notable *in vivo* protective action and a notable dose-effect relationship. Meanwhile, the drug efficacy of the 0.9mg/kg peramivir DPI was superior to that of the peramivir sodium chloride injection (10mg/kg) and the oseltamivir phosphate capsule (10mg/kg).

Experiment 5

**Impact of Nebulized Administration of Peramivir DPI on Pulmonary System Virus Titer of Mice Inflected with Influenza A/PR/8/34 Virus**

84 SPF Balb/c mice, half male and half female, weighing 21.2~26.6g each, were stochastically divided into 7 groups: a normal control group, a virus model group, an oseltamivir phosphate capsule group (Tamiflu) (10mg/kg), a peramivir I.V. group (10mg/kg), and peramivir dry powder inhaler (example 3) groups I-III (0.1, 0.3, 0.9mg/kg), twelve mice in each group (in each group, F04-F06 and M04~M06 were subjected to 24h hemagglutination titer measurement, and F01~F03 and M1~M03 were subjected to 48h hemagglutination titer measurement). The mice in each group were mildly anaesthetized with diethyl ether and intranasally administered with influenza A/PA/8/34 virus liquid (5 LD$_{50}$ diluted solution resulting from diluting the influenza virus bulk with a 4°C precooled blank culture medium), 60$\mu$L per mouse, wherein the normal control group was intranasally administered with the blank culture medium of the same volume. Prior to daily administration, the peramivir I.V. injection was prepared into a 1.0mg/mL liquor with 0.9% sodium chloride injection, and the oseltamivir phosphate capsule was prepared into a 0.5mg/mL liquor with pure water. The mice in each group were all administered at 2h post- modeling of the intranasal virus infection, wherein the mice in each peramivir DPI group were administered with a corresponding dosage of peramivir dry powder inhaler, the mice in the peramivir intravenous injection group were intravenously injected with the peramivir liquid as per a dosage of 10mL/kg, the oseltamivir phosphate capsule group were intragastrically administered with oseltamivir phosphate liquid as per a dosage of 20mL/kg, and the normal control group and the model control group were sprayed with air of the same volume via their trachea. The mice were administered once per day, 2 days in succession.

6 mice, half male and half female, were stochastically selected from each group at 24h and 48h post- administration, respectively. The mice were euthanized by cervical dislocation and their lungs were procured through dissection and then put in a homogenizer. 0.9% sodium chloride injection was added according to a ratio of the lung weight (g) to 0.9% sodium chloride injection (mL) = 1:9, followed by grinding the homogenates into mouse lung suspensions. The mouse lung suspensions were subjected to centrifugation at 2500rpm for 20min under a 4°C environment, with the supernatant being acquired for future use. Except the first column, 50$\mu$L PBS (Phosphate Buffer Saline) was added into hemagglutination plate wells. 100$\mu$L to-be-tested supernatant was added into each well in the first column, respectively, followed by adding with serial dilution till the wells in the 11th column, and then 50$\mu$L 0.5% chicken erythrocyte was added into each well, respectively, followed by setting for 40min; the hemagglutination plate was then tilted to observe whether the erythrocyte flew in a teardrop shape. The hemagglutination titer was represented by a logarithmic value of the maximum dilution factor completely inducing coagulation of the chicken erythrocyte, wherein the higher the hemagglutination titer, the higher the titer of the virus in the mouse lung suspension.

Table 12 Impact of Peramivir DPI on Viral Titer of Mouse Lung Tissue Infected with Influenza A/PR/8/34 Virus ( $\bar{x} \pm s$ , n=6)

| Group # | Dosage (mg/kg) | hemagglutination titer | |
|---|---|---|---|
| | | 24h | 48h |
| Normal control group | / | 0.0±0.0 | 0.0±0.0 |
| Virus control group | / | **4.7±1.2++** | **5.8±1.2++** |
| Oseltamivir phosphate group | 10 | **2.5±0.5**** | **2.2±0.8**** |
| Peramivir I.V. injection group | 10 | **2.2±1.0**** | **1.5±0.5**** |
| Peramivir DPI group I | 0.1 | **3.2±0.8**** | **2.5±0.8**** |
| Peramivir DPI group II | 0.3 | **1.8±1.2**** | **1.7±0.8**** |
| Peramivir DPI group III | 0.9 | **1.3±0.5**** | **1.2±0.4**** |
| Note: compared with the normal control group ++$P \leq 0.01$; compared with the virus control group, **$P \leq 0.01$ | | | |

As shown in Table 12, the mice in the normal control group were not infected by the virus such that their hemagglutination titers were 0. Compared with the normal control group, the lung hemagglutination titer of the virus control group were notably increased ($P \leq 0.01$) at 24h and 48h post-infection. Compared with the virus control group, the lung hemagglutination titers of the oseltamivir phosphate group, the peramivir I.V. injection group, and the peramivir inhaler groups I~III were all notably decreased ($P \leq 0.01$) at 24h and 48h post-infection. The results showed that administration of peramivir DPI at 2h post-infection can effectively decrease the titer of the influenza A virus in the lungs of mice, indicating that it has a significant antivirus effect and a notable dose-effect relationship; meanwhile, the drug efficacy of the 0.9mg/kg peramivir DPI was superior to that of the peramivir sodium chloride injection (10mg/kg) and the oseltamivir phosphate capsule (10mg/kg).

What have been described above are only preferred embodiments of the present disclosure, not intended for limiting the substantive scope of the present disclosure. The substantive technical content of the present disclosure is broadly defined in the appended claims. Any technical entity or method accomplished by others with a completely identical scope as defined in the appended claims is an equivalent modification to the present disclosure, which shall fall into the scope of the appended claims of the disclosure.

**Claims**

1. A peramivir dry powder inhaler, comprising: dry powder of a peramivir active pharmaceutical ingredient API or an acceptable salt or hydrate of the peramivir API, wherein a dry powder loading capacity of a single-dose preparation ranges from 5 to 30mg; and particle size distribution D10 of the dry powder ranges from 1.3$\mu$m to 3$\mu$m, particle size distribution D50 of the dry powder ranges from 3$\mu$m to 6$\mu$m, and particle size distribution D90 of the dry powder ranges from 6 to 13$\mu$m.

2. The peramivir dry powder inhaler according to claim 1, wherein the dry powder has an angle of repose ranging from 33° to 37°, preferably 34°, 35°, 36°; a bulk density of 0.23~0.28g/cm$^3$, preferably 0.24, 0.25, 0.26, 0.27g/cm$^3$; and a tapped density of 0.39~0.44g/cm$^3$, preferably 0.40, 0.41, 0.42, 0.43g/cm$^3$.

3. The peramivir dry powder inhaler according to claim 1, wherein the Carr's flowability index of the dry powder ranges from 58 to 65, preferably 59, 60, 61, 62.

4. The peramivir dry powder inhaler according to claim 1, wherein the percentage of the dry powder with a fine powder fraction FPF less than 4.46μm is greater than 30% and less than 45%, preferably 32%, 33%, 34%, 35%, 36%, 37%, 38%, 40%, 41%, more preferably ranging from 34% to 38%.

5. The peramivir dry powder inhaler according to claim 1, wherein the dry powder has an aerodynamic particle size greater than 2.5μm and less than 3.6μm, preferably ranging from 2.8μm to 3.5μm or from 2.6μm to 3.1μm or from 2.9μm to 3.4μm, and more preferably from 3.0μm to 3.2μm or from 3.1μm to 3.3μm.

6. The peramivir dry powder inhaler according to claim 1, wherein the particle size distribution D10 of the dry powder ranges from 1.3μm to 2.0μm, preferably 1.4μm, 1.5μm, 1.6μm, 1.7μm, 1.8μm, 1.9μm, 2.1μm; the particle size distribution D50 is preferably 3.5μm, 4.5μm, 5μm, 5.5μm; and the particle size distribution D90 is preferably 6.2μm, 6.5μm, 7μm, 7.5μm, 7.8μm, 8.0μm, 8.4μm, 9.0μm, 10μm, 11μm, 12μm.

7. The peramivir dry powder inhaler according to claim 1, wherein the dry powder inhaler is prepared by a peramivir trihydrate.

8. A peramivir dry powder inhaler preparation, wherein the dry powder according to claim 1 is prepared into a capsule dosage form, a niosome dosage form, or a depot dosage form, wherein a dry powder loading capacity of each unit of dosage form ranges from 5 to 30mg, preferably 20mg.

9. A method of preparing the peramivir dry powder inhaler according to claim 1, comprising: jet milling a peramivir trihydrate at a feed pressure 0.4-0.55MPa and a milling pressure 0.4~0.6MPa, whereby to obtain a dry powder.

## INTERNATIONAL SEARCH REPORT

| International application No. |
| --- |
| **PCT/CN2021/080768** |

| A. | CLASSIFICATION OF SUBJECT MATTER |
| --- | --- |

A61K 9/72(2006.01)i; A61K 9/14(2006.01)i; A61K 31/196(2006.01)i; A61P 31/16(2006.01)i

According to International Patent Classification (IPC) or to both national classification and IPC

| B. | FIELDS SEARCHED |
| --- | --- |

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS; CNTXT; DWPI; SIPOABS; WOTXT; USTXT; EPTXT; CNKI; 万方; 读秀; STN; ISI_Web of Science; Elsevier Science; PubMed; Wiley InterScience; SpringerLink; NCBI: 南鑫, 帕拉米韦, 330600-85-6, 1041434-82-5, 吸入, 粉, 粒径, 粒度, 大小, 分布, D10, D50, D90, 休止角, 松密度, 振实密度, 流动指数, 气流粉碎, Nucien, peramivir, inhale, powder, particle size, distribution, angle of repose, bulk density, tap density, flow index, jet pulverization

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| PX | CN 111358773 A (GUANGZHOU NANXIN PHARMACEUTICAL CO., LTD.) 03 July 2020 (2020-07-03) claims 1-8, description, paragraphs [0015]-[0027] | 1-9 |
| X | CN 106420621 A (MOBAIRUI CHENGDU BIOLOGICAL TECHNOLOGY CO., LTD.) 22 February 2017 (2017-02-22) description, paragraphs [0003]-[0013] | 1-9 |
| A | CN 103446051 A (CHEN, Yongqi et al.) 18 December 2013 (2013-12-18) entire document | 1-9 |
| A | WO 2009143011 A1 (NOVARTIS AG. et al.) 26 November 2009 (2009-11-26) entire document | 1-9 |
| A | CN 102573886 A (MUTUAL PHARMACEUTICAL COMPANY INC.) 11 July 2012 (2012-07-11) entire document | 1-9 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **14 May 2021** | **03 June 2021** |

| Name and mailing address of the ISA/CN | Authorized officer |
| --- | --- |
| **China National Intellectual Property Administration (ISA/CN)** **No. 6, Xitucheng Road, Jimenqiao, Haidian District, Beijing 100088 China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2021/080768**

| C. | DOCUMENTS CONSIDERED TO BE RELEVANT | |
|---|---|---|
| Category* | Citation of document, with indication, where appropriate, of the relevant passages | Relevant to claim No. |
| A | CN 109953977 A (SHENZHEN HUALIKANG BIO-MEDICINE CO., LTD. et al.) 02 July 2019 (2019-07-02)<br>entire document | 1-9 |
| A | 郭娜 等 (GUO, Na et al.). "扎那米韦吸入粉雾剂理化性质及其初步稳定性考察 (Physicochemical Property and Stability of Zanamivir Dry Powder Inhalation)"<br>中国药学杂志 (Chinese Pharmaceutical Journal),<br>Vol. 47, No. 23, 31 December 2012 (2012-12-31),<br>pp. 1911-1914 | 1-9 |
| A | 彭继千 等 (PENG, Jiqian et al.). "扎那米韦鼻用粉雾剂粉体性质研究 (Study on Powder Characterization of Zanamivir Nasal Powder)"<br>中国现代应用药学 (Chinese Journal of Modern Applied Pharmacy),<br>Vol. 35, No. 10, 31 October 2018 (2018-10-31),<br>pp. 1462-1465 | 1-9 |
| A | CN 104487075 A (PULMATRIX, INC.) 01 April 2015 (2015-04-01)<br>entire document | 1-9 |

Form PCT/ISA/210 (second sheet) (January 2015)

INTERNATIONAL SEARCH REPORT
Information on patent family members

International application No.

**PCT/CN2021/080768**

| Patent document cited in search report | | | Publication date (day/month/year) | Patent family member(s) | | | Publication date (day/month/year) |
|---|---|---|---|---|---|---|---|
| CN | 111358773 | A | 03 July 2020 | None | | | |
| CN | 106420621 | A | 22 February 2017 | None | | | |
| CN | 103446051 | A | 18 December 2013 | WO | 2015027848 | A1 | 05 March 2015 |
| WO | 2009143011 | A1 | 26 November 2009 | None | | | |
| CN | 102573886 | A | 11 July 2012 | US | 2011229437 | A1 | 22 September 2011 |
| | | | | EP | 2477642 | A4 | 13 March 2013 |
| | | | | WO | 2011035128 | A1 | 24 March 2011 |
| | | | | EP | 2477642 | A1 | 25 July 2012 |
| CN | 109953977 | A | 02 July 2019 | None | | | |
| CN | 104487075 | A | 01 April 2015 | JP | 2015509788 | A | 02 April 2015 |
| | | | | US | 2020261667 | A1 | 20 August 2020 |
| | | | | JP | 2019181244 | A | 24 October 2019 |
| | | | | CN | 109999013 | A | 12 July 2019 |
| | | | | NZ | 629722 | A | 31 March 2017 |
| | | | | CA | 2865972 | A1 | 06 September 2013 |
| | | | | US | 10806871 | B2 | 20 October 2020 |
| | | | | CN | 107596518 | A | 19 January 2018 |
| | | | | WO | 2013130767 | A1 | 06 September 2013 |
| | | | | AU | 2013225982 | B2 | 30 November 2017 |
| | | | | US | 2017304564 | A1 | 26 October 2017 |
| | | | | US | 2015136130 | A1 | 21 May 2015 |
| | | | | US | 10589039 | B2 | 17 March 2020 |
| | | | | EP | 2819672 | A1 | 07 January 2015 |
| | | | | AU | 2013225982 | A1 | 18 September 2014 |
| | | | | JP | 2017140515 | A | 17 August 2017 |
| | | | | HK | 1204989 | A0 | 11 December 2015 |
| | | | | IN | 201406933 | P1 | 31 August 2016 |
| | | | | IL | 234153 | A1 | 30 October 2014 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• CN 109771398 B